# EUROPEAN PATENT SPECIFICATION

(11) **EP 3 945 529 B1**
(45) Date of publication and mention of the grant of the patent: **09.07.2025**
(21) Application number: 21187410.2
(22) Date of filing: 23.07.2021
(51) Int. Cl.: G16H 50/30, G16H 40/63

(54) **STROKE SCALE ASSESSMENT**
SCHLAGANFALLSKALENBEURTEILUNG
ÉVALUATION D'ÉCHELLE D'AVC

(30) Priority: 27.07.2020 US 202063056750 P
(43) Date of publication of application: 02.02.2022
(73) Proprietor: Welch Allyn, Inc., Skaneateles Falls, NY 13153-0220 (US)
(72) Inventor: BURKARSKI, Megan M., New York, 13153 (US); DETOR, Mia, New York, 13153 (US); MCSWEENEY, WonKyung, New York, 13153 (US); LIA, Raymond A., New York, 13153 (US)
(74) Representative: Reddie & Grose LLP

(56) References cited:
- US-A1- 2007 157 385
- US-A1- 2016 042 123
- US-A1- 2017 007 167
- ANONYMOUS: "National Institutes of Health Stroke Scale (NIHSS)", WIKIPEDIA, 28 June 2020 (2020-06-28), pages 1 - 14, XP055872340, Retrieved from the Internet <URL:https://en.wikipedia.org/w/index.php?title=National_Institutes_of_Health_Stroke_Scale&oldid=964857830> [retrieved on 20211213]

## Description

A stroke is a sudden loss of brain function caused by interruption or loss of blood flow to the brain or rupture of blood vessels in the brain that results in injury or death of brain tissue. There are two main types of stroke. An ischemic stroke is due to lack of blood flow typically caused by a blood clot that blocks or plugs a blood vessel in the brain. A hemorrhagic stroke occurs when blood from an artery bleeds into the brain such as when a weakened blood vessel ruptures, and pressure from the leaked blood damages brains cells, and as a result, the damaged area is unable to function properly. Both types of stroke result in parts of the brain not functioning properly. Stroke symptoms may include an inability to move or feel on one side of the body, problems understanding or speaking, dizziness, or loss of vision on one side.

Health care providers often use the National Institute of Health Stroke Scale (NIHSS) to assess stroke symptoms, see https://www.ninds.nih.gov/Disorders/All-Disorders/Stroke-Information-Page and also https://www.stroke.nih.gov/documents/NIH_Stroke_Scale_508C.pdf for a chart used to conduct the NIH stroke scale. The scale is typically performed several times per day while a stroke patient is hospitalized. Although the scale is designed to objectively quantify stroke symptoms, at least some of the individual components that make up the scale are subjective such that the results can differ between clinicians. Additionally, the tasks that are needed to complete the stroke scale require a clinician's direct involvement and are time consuming.

US 2016/042123 A1 discloses methods and systems for the monitoring and assessment of clinical function through sensors and interactive patient responses (in particular, for patients with stroke).

In general terms, the present disclosure relates to automated stroke assessment. In one possible configuration, a system and method for automated stroke assessment provide a technical effect by mitigating clinician intervention and subjectivity. Various aspects are described in this disclosure, which include, but are not limited to, the following aspects.

The invention is defined by the appended independent claims.

The invention will now be further described by way of example with reference to the accompanying drawings, in which:
FIG. 1 schematically illustrates a healthcare facility that includes a stroke scale system that determines a stroke scale score for a patient.
FIG. 2 schematically illustrates the stroke scale system of FIG. 1.
FIG. 3 illustrates a patient support apparatus of the stroke scale system of FIG. 2.
FIG. 4 schematically illustrates the patient support apparatus of FIG. 3.
FIG. 5 schematically illustrates a display unit of the stroke scale system of FIG. 2.
FIG. 6 schematically illustrates an audio unit of the stroke scale system of FIG. 2.
FIG. 7 schematically illustrates motion detectors of the stroke scale system of FIG. 2.
FIG. 8 illustrates a method for determining a stroke scale score for a patient positioned on a patient support apparatus.
FIG. 9 illustrates a series of stroke scale examinations performed in the method of FIG. 8.
FIG. 10 illustrates details of a level of consciousness inquiry performed in the method of FIG. 9.
FIG. 11 illustrates details of a level of consciousness physical examination performed in the method of FIG. 9.
FIG. 12 illustrates details of a gaze tracking examination performed in the method of FIG. 9.
FIG. 13 illustrates details of a visual field examination performed in the method of FIG. 9.
FIG. 14 illustrates details of a facial palsy examination performed in the method of FIG. 9.
FIG. 15 illustrates details of an arm physical examination performed in the method of FIG. 9.
FIG. 16 illustrates details of a leg physical examination performed in the method of FIG. 9.
FIG. 17 illustrates details of a limb ataxia examination performed in the method of FIG. 9.
FIG. 18 illustrates details of a sensory stimulus examination performed in the method of FIG. 9.
FIG. 19 illustrates details of a comprehension examination performed in the method of FIG. 9.
FIG. 20 illustrates details of a dysarthria examination performed in the method of FIG. 9.
FIG. 21 shows an image, a naming sheet, and a list of sentences displayed during the comprehension examination of FIG. 19.
FIG. 22 shows exemplary words displayed during the dysarthria examination of FIG. 20.
FIG. 23 schematically illustrates an exemplary architecture of a computing device that can be used to implement aspects of the present disclosure.
FIG. 24 schematically illustrates an exemplary siderail of the patient support apparatus of FIGS. 3 and 4 that can be used to implement aspects of the present disclosure.

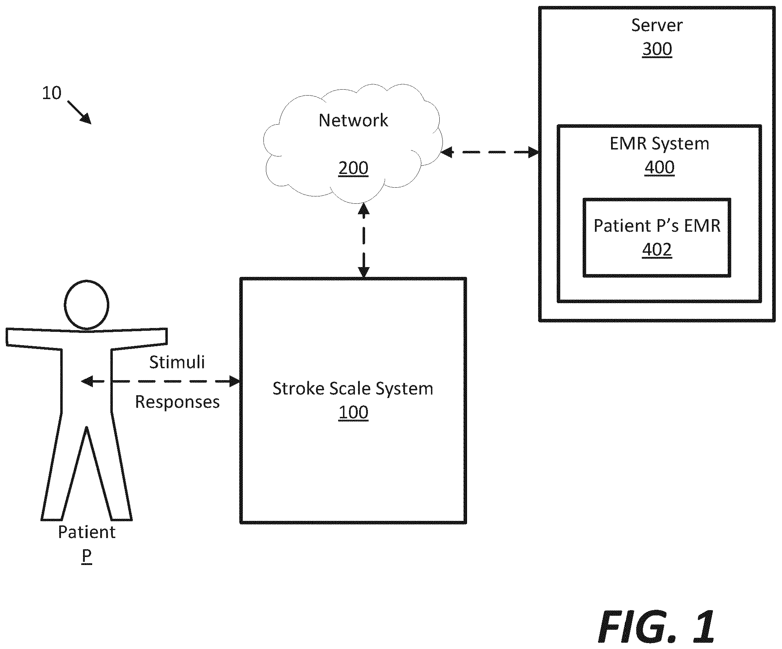

FIG. 1 schematically illustrates a healthcare facility 10 that includes a stroke scale system 100. The stroke scale system 100 can determine a stroke scale score for a patient P located in the healthcare facility 10. Advantageously, the stroke scale score can be determined through an automated method that mitigates human error and subjectivity. Illustrative examples of the healthcare facility 10 may include, without limitation, a hospital, a medical clinic, a long-term-care facility, a nursing home, a skilled nursing facility, a surgical center, a physician's office, and may include the patient P's home.

The stroke scale system 100 communicates with the patient P such as by instructing the patient P to perform one or more tasks. Additionally, the stroke scale system 100 can provide various stimuli to induce a response from the patient P. Advantageously, the stroke scale system 100 can provide the instructions and stimuli to the patient P without requiring any intervention from a clinician or staff member of the healthcare facility 10.

The stroke scale system 100 records the patient P's performance of the instructions and the patient P's responses to the stimuli, and uses the recorded performance and responses to objectively assess and score various stroke symptoms. Thereafter, the stroke symptom scores are used to determine a stroke scale score for the patient P. Illustrative examples of the stroke symptoms assessed by the stroke scale system 100 include, without limitation, the patient P's level of consciousness, vision including gaze and visual field, facial palsy, motor control of arms and legs, limb ataxia, sense of touch, comprehension, speech, and attention.

In certain examples, the stroke scale system 100 determines a stroke scale score for the patient P by combining the stroke symptom scores, and utilizes a network 200 to transfer the stroke scale score to a server 300 that is remotely located with respect to the Patient P and stroke scale system 100. In alternative examples, the stroke scale system 100 does not determine the individual stroke symptom scores or the stroke scale score, and instead transfers to the server 300 data collected from the patient P's performance of the instructions and responses to stimuli, and the server 300 determines the stroke symptom scores, and combines the stroke symptom scores to determine the stroke scale score for the patient P.

The network 200 can include any type of wired or wireless connections or any combinations thereof. Examples of wireless connections include digital cellular network connections such as 5G. In some examples, wireless connections can be accomplished using, without limitation, Bluetooth, Wi-Fi, RFID, NFC, ZigBee, and the like.

In certain examples, the server 300 includes an electronic medical record system 400 (alternatively termed electronic health record, EMR/EHR). Advantageously, the server 300 can automatically store the stroke scale score of the patient P in an electronic medical record 402 or electronic health record of the patient P located in the EMR system 400 without requiring any input or intervention from a clinician. Advantageously, this can further reduce human errors that may result from manually updating the electronic medical record of the patient P.

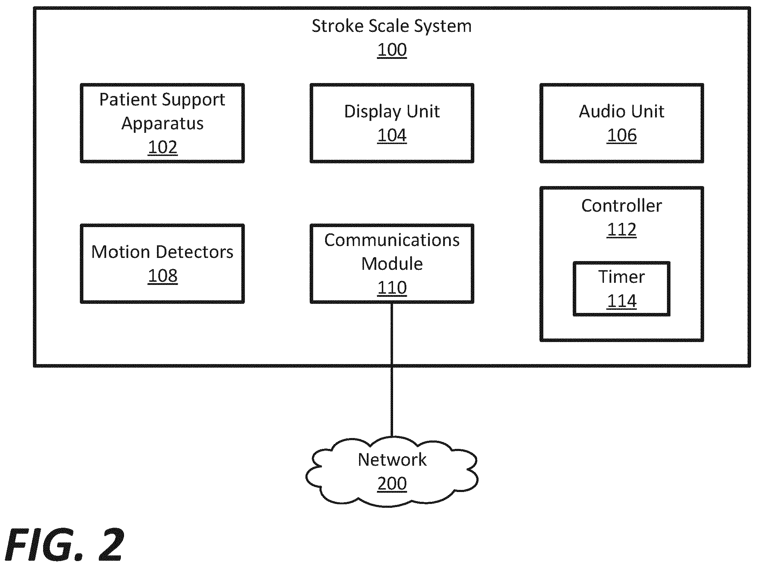

FIG. 2 schematically illustrates the stroke scale system 100. The stroke scale system 100 includes a patient support apparatus 102, a display unit 104, an audio unit 106, motion detectors 108, a communications module 110, and a controller 112.

The controller 112 is operatively coupled to each of the patient support apparatus 102, display unit 104, audio unit 106, motion detectors 108, and communications module 110. The controller 112 is configured to control and coordinate the operation of each of these components to perform an automated method to determine the stroke scale score of the patient P. The controller 112 is a computing device. In certain examples, the controller 112 includes a timer 114 that can be used to automatically initiate a method for determining a stroke scale score when a clinician in the healthcare facility 10 is not available to initiate the method.

The communications module 110 is connected with the network 200 such that is able to transfer the stroke scale score and/or the recorded performance of the instructions and responses to the stimuli to another device or system, such as the server 300.

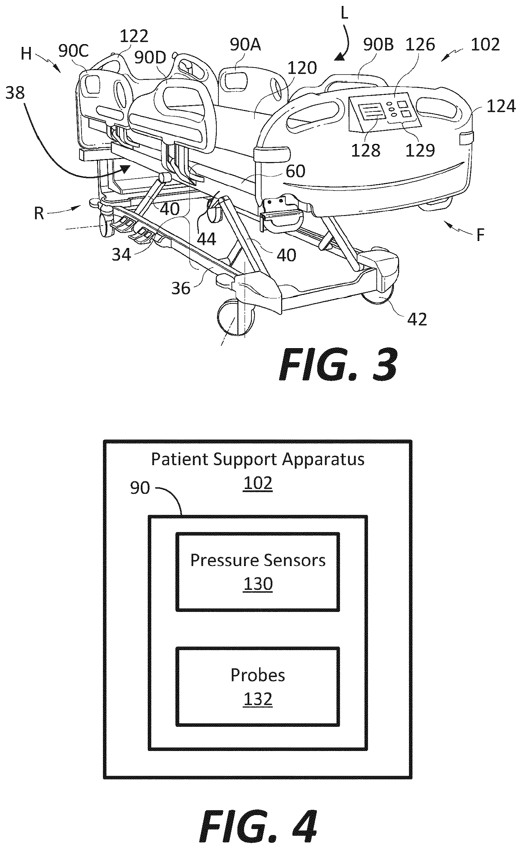

FIG. 3 illustrates an example the patient support apparatus 102. While FIG. 3 depicts the patient support apparatus 102 as a hospital bed, alternative examples are possible where the patient support apparatus 102 may be a chair, a recliner, surgical table, or any other type of support apparatus. Thus, the description provided herein is not limited to hospital beds.

The patient support apparatus 102 extends longitudinally from a head end H to a foot end F and laterally from a left side L to a right side R, where left and right are taken from the perspective of a supine occupant of the patient support apparatus 102. The patient support apparatus 102 includes a frame 34 that has a base frame 36 and an elevatable frame 38 that is supported on the base frame 36 by supports 40. The elevatable frame 38 is vertically moveable relative to the base frame 36. The frame 34 includes wheels 42 extending from the base frame 36 to the floor to facilitate the portability of the bed around the healthcare facility 10.

The elevatable frame 38 includes a sub-frame 44 and a deck 60 that supports a mattress 120. The mattress 120 is flexible such that it conforms to the profile of the deck 60 as the orientation of the deck 60 is adjusted between horizontal and vertical orientations.

The patient support apparatus 102 includes a left siderail assembly having at least one left siderail mounted on the left side of the frame and a right siderail assembly having at least one right siderail mounted on the right side of the frame. In the example of FIG.3, the left siderail assembly includes an upper left siderail 90A and a lower left siderail 90B, and the right siderail assembly includes an upper right siderail 90C and a lower right siderail 90D.

In certain examples, the upper siderails 90A, 90C are connected to an upper body section of the deck 60 and rotate with the upper body section as that section rotates, while the lower siderails 90B, 90D are connected to a portion of the elevatable frame 38 that does not rotate with respect to the sub-frame 44. Accordingly, the lower siderails 90B, 90D are always at a fixed orientation relative to sub-frame 44 as shown in FIG. 3.

Each siderail 90A-90D is positionable at a deployed position at which its upper edge is higher than the top of the mattress 120 and at a stowed position at which its upper edge is lower than the top of the mattress 120. When the deployed position, the siderail prevents the patient P from exiting the patient support apparatus 102. When in the stowed position, the siderail allows the patient P to enter and exit the patient support apparatus 102. In some examples, the siderails 90A-90D are also positionable at intermediate positions that are not as high as the deployed position nor as low as the stowed position. In the example illustrated in FIG. 3, all four siderails 90A-90D are in the deployed position.

The patient support apparatus 102 includes a headboard 122 and a footboard 124. In certain examples, the footboard 124 is removable from the foot end F of the frame 34 in order to accommodate occupant egress from the foot end F. For example, in certain examples, the patient support apparatus 102 can be adjusted so that its profile mimics that of a chair. When the patient support apparatus 102 is in a chair-like profile, the footboard 124 can be removed to facilitate egress and ingress at the foot end F of the patient support apparatus 102.

The patient support apparatus 102 can further include a user interface 126 for operation by a clinician. The user interface 126 includes a display 128 for displaying information, and user input devices 129 such as buttons, switches, or a keyboard. In the example illustrated in FIG. 3, the user interface 126 is positioned on the footboard 124.

FIG. 4 schematically illustrates the patient support apparatus 102. Referring now to FIGS. 3 and 4, the patient support apparatus 102 includes one or more pressure sensors 130 provided on a portion of the patient support apparatus 102 that can be grasped by the patient P. In examples where the patient support apparatus 102 is a hospital bed, such as in the example depicted in FIG. 3, the pressure sensors 130 are provided on at least one siderail 90 of the bed. In alternative examples, for example when the patient support apparatus 102 is a chair, the pressure sensors 130 are provided on an armrest of the chair.

In alternative examples, the pressure sensors 130 can be provided on a device that is separate from the patient support apparatus 102, and that is accessible by the patient P such that the patient P can grasp the device while being supported on the patient support apparatus 102. The device may or may not attach to the patient support apparatus 102. In such alternative examples, the device can communicate with the communications module 110 to transfer data detected by the pressure sensors 130.

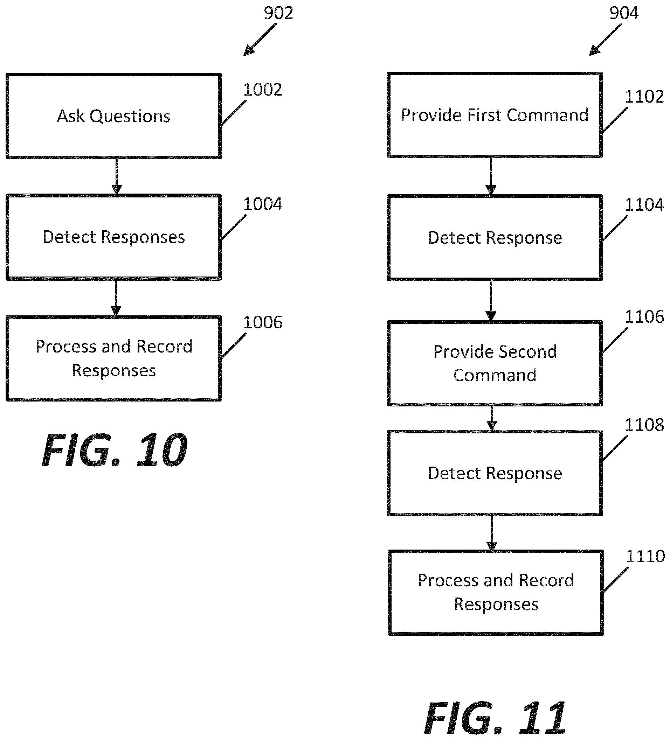

As will be described in more detail, a level of consciousness (LOC) physical exam 904 (see FIG. 11) is performed as part of a method for determining the stroke scale score of the patient P, and includes instructions for the patient P to grasp a portion of the patient support apparatus 102 such as a siderail of a hospital bed or an armrest of a chair, or alternatively the instructions may instruct the patient P to grasp a device separate from the patient support apparatus 102 which may or may not be attached to the patient support apparatus 102. The pressure sensors 130 are used by the stroke scale system 100 to detect the patient P's response to the instructions by measuring an applied pressure. Advantageously, the pressure sensors 130 can objectively measure the strength of the pressure applied by the patient P to remove subjectivity from the stroke scale score determination.

The patient support apparatus 102 further includes one or more probes 132 that provide a sensory stimulus that can be felt by the patient P such as by gently poking or prodding the patent P without causing harm to the patient P. The probes 132 are embedded in a siderail of the patient support apparatus 102 adjacent to the pressure sensors 130.

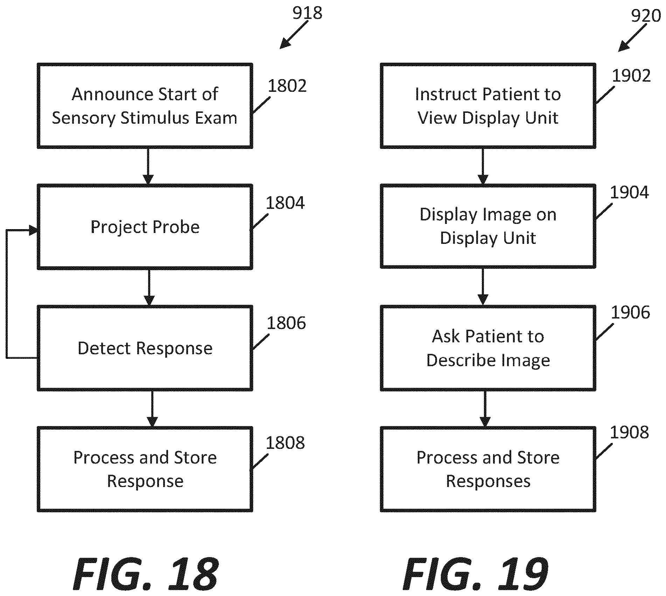

As will be described in more detail, a sensory stimulus exam 918 (see FIG. 18) is performed as part of a method for determining the stroke scale score of the patient P. The sensory stimulus exam 918 uses the probes 132 to provide sensory stimuli to the patient P, and the stroke scale system 100 detects and/or measures the patient P's response to the stimuli.

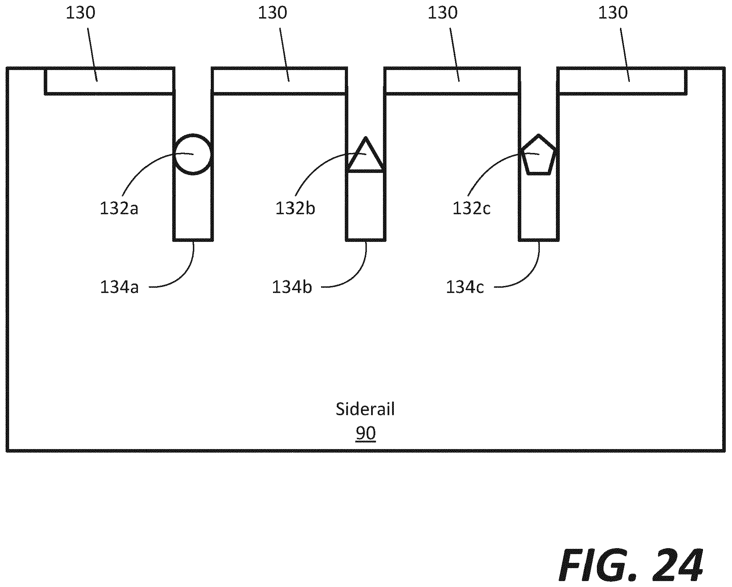

FIG. 24 schematically illustrates an example of a siderail 90 of the patient support apparatus 102 that can be used to implement aspects of the present disclosure. As described above, the patient support apparatus 102 can include at least one pressure sensor 130 on a portion of the siderail 90 that can be grasped by the patient P such that the pressure sensor 130 can detect when the patient P grasps the siderail 90 and can measure the pressure applied by the patient P to the siderail 90 in response to a command that requests the patient P to grasp the siderail 90.

As further shown in FIG. 24, one or more probes 132a-132c are embedded in the siderail 90 adjacent to the location of the pressure sensor 130. The probes 132a-132c are stored inside respective cavities 134a-134c in the siderail 90, and are controlled by the controller 112 to project outwardly to provide sensory stimuli during the sensory stimulus exam 918. After completion of the sensory stimulus exam 918, the controller 112 instructs the probes 132a-132c to return inside their respective cavities 134a-134c so that they do not disturb the patient P.

As shown in FIG. 24, multiple probes 132a-132c can be used by the stroke scale system 100 to provide different types of sensory stimuli. For example, probe 132a, which is depicted as having a blunt distal end, can be used to provide a mild sensory stimulus, while probe 132c has a pointed distal end that can be used to provide a moderate sensory stimulus, and a probe 132b includes another pointed distal end that can be used to provide a stronger sensory stimulus. While multiple probes are shown in the example of the siderail 90 shown in FIG. 24, in alternative examples, the siderail 90 may include only one probe, may include two probes, or may include more than three probes. Thus, FIG. 24 is provided by way of illustration only.

The arrangement depicted in FIG. 24 can be used on multiple siderails 90 of the patient support apparatus 102 such as in the left siderail assembly that includes the upper left siderail 90A and the lower left siderail 90B, and the right siderail assembly includes the upper right siderail 90C and the lower right siderail 90D. Advantageously, the sensory stimuli from the probes 132a-132c can be provided to both the left and right sides of the patient P's body. Additionally, the pressure sensors 130 can be used to determine whether the patient P is able to grasp a siderail on the left siderail assembly or on the right siderail assembly.

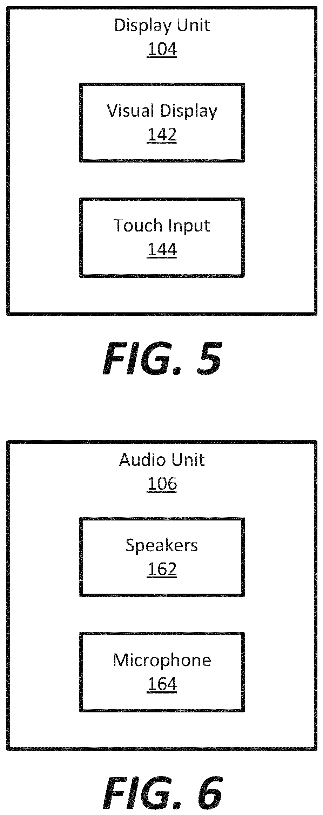

FIG. 5 schematically illustrates the display unit 104. The display unit 104 displays various objects for viewing by the patient P during the automated method. Additionally, in scenarios where the patient P is deaf or has hearing impairment, text and visual instructions can be presented on the display unit 104 instead of audio instructions.

In certain examples, the display unit 104 is both an input and output device such as a touchscreen that has both a visual display 142 and a touch input 144. In such examples, the display unit 104 can be fixed to the patient support apparatus 102 such that the visual display 142 can be both viewed and touched by the patient P to enter one or more responses during the method for determining the stroke scale score. As an illustrative example, the display unit 104 can be attached to a siderail 90 of the patient support apparatus 102.

In some examples, the display unit 104 is only an output device such that it includes the visual display 142, but not the touch input 144. In such examples, the display unit 104 does not need to be in close proximity with the patient P because there is no need for the patient P to touch and interact with the display unit 104. Thus, the display unit 104 can be fixed to the footboard 124 of the patient support apparatus 102 so that it can be easily viewed by the patient P when sitting upright on the patient support apparatus 102. Also, in such examples, the display unit 104 does not need to be fixed to the patient support apparatus 102 such that the display unit 104 can be mounted on a portable stand that can be positioned adjacent to or in front of the patient support apparatus 102, or can be mounted to a wall adjacent to or in front of the patient support apparatus 102 for viewing by the patient P while on the support apparatus.

FIG. 6 schematically illustrates the audio unit 106 as including one or more speakers 162 and a microphone 164. The speakers 162 are used by the stroke scale system 100 to provide audible instructions to the patient P during the method for determining the stroke scale score. For example, the audible instructions can include asking the patient P the month and his or her age, to open and close their eyes, grip, and release a hand, follow with their eyes the movement of an object displayed on the display unit 104, and so on. Additionally, the speakers 162 can be used to announce the start of the method for determining the stroke scale score. In scenarios where the patient P is deaf, instead of using the audio unit 106 to present audible instructions, the stroke scale system 100 can display text and visual instructions on the display unit 104.

The microphone 164 is used by the stroke scale system 100 to record audible responses from the patient P to the instructions and stimuli that are provided by the stroke scale system 100. For example, the microphone 164 can be used to record the patient P's responses to a level of consciousness inquiry that asks the patient P to state the month and his or her age.

Additionally, the audio unit 106 can provide the audio instructions and information depending on the preferred language of the patient P, and the microphone 164 can record the responses from the patient P in their preferred language. Thus, the stroke scale system 100 is multilingual. In certain examples, the preferred language of the patient P is identified from the electronic medical record 402 of the patient P stored in the EMR system 400 (see FIG. 1).

In certain examples, the display unit 104 and audio unit 106 are packaged together in a single device such as a monitor, tablet computer, and the like. Alternatively, the display unit 104 and audio unit 106 can be contained in separate devices.

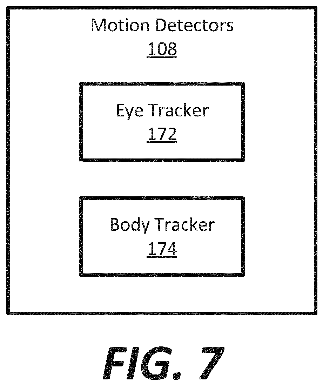

FIG. 7 schematically illustrates the motion detectors 108 of the stroke scale system 100. The motion detectors 108 detect movements of the patient P in response to the instructions and stimuli that are provided for determining the stroke scale score. The motion detectors 108 are used by the stroke scale system 100 to objectively identify compliance or non-compliance with the instructions and stimuli such that the motion detectors 108 mitigate clinician subjectivity. Additionally, the motion detectors 108 provide improved refinement and precision in quantifying the patient P's compliance or non-compliance with the instructions and stimuli.

In the example depicted in FIG. 7, the motion detectors 108 include an eye tracker 172 for measuring the eye movement of the patient P during the automated method. For example, the eye tracker 172 can measure the point of gaze of the patient P when the patient P is instructed to follow a moving target displayed on the display unit 104. The eye tracker 172 is used by the stroke scale system 100 to record the eye movement and gaze of the patient P.

The eye tracker 172 can include a camera that captures images of the patient P's eyes from which eye position and movement are extracted. In certain examples, the eye tracker 172 uses infra-red eye tracking such as by detecting infra-red light reflection from the patient P's eyes using the camera or some other optical sensor. The detected infra-red light reflection is analyzed to extract the patient P's eye position and movement.

The motion detectors 108 can further include a body tracker 174 that detects the position and movement of the patient P's limbs. For example, the method for determining the stroke scale score can include instructing the patient P to cover one eye with their hand, and the body tracker 174 can be used by the stroke scale system 100 to confirm whether the correct eye has been covered. Additionally, the body tracker 174 can detect facial expressions of the patient P in response to the instructions or stimuli provided during the method.

As described above, the method for determining the stroke scale score of the patient P can include instructions for the patient P to grab a portion of the patient support apparatus 102 such as a siderail of a hospital bed or an armrest of a chair. In certain examples, the body tracker 174 can be used by the stroke scale system 100 to detect whether the patient P grabbed a portion of the patient support apparatus 102 such that the body tracker 174 can be used to determine compliance with the instructions instead of the pressure sensors 130.

In certain examples, the body tracker 174 uses radar technology to detect the position and movement of the patient P's limbs. In such examples, the body tracker 174 transmits electromagnetic wave signals that the patient P's limbs reflect. By capturing the reflected signal, the body tracker 174 can determine the position and movement of the limbs of the patient P. In some examples, the body tracker 174 uses millimeter waves (also referred to as mmWaves) which is a special class of radar technology that uses short-wavelength electromagnetic waves. In alternative examples, other technologies can be used by the body tracker 174 to track the position and movement of the patient P's limbs such as a camera that captures images from which limb position and movement are extracted.

In certain examples, the body tracker 174 is mounted to the patient support apparatus 102 such as by attachment to a siderail 90 or the footboard 124 of the apparatus. In other examples, the body tracker 174 is fixed to a ceiling above the patient support apparatus 102 or to a wall that is adjacent to or in front of the patient support apparatus 102. In some examples, the body tracker 174 is mounted to a portable stand that can be positioned adjacent to or in front of the patient support apparatus 102.

In certain examples, the functions of both eye tracker 172 and body tracker 174 are performed by a single device. As an illustrative example, a radar system such as one that uses mmWaves can be used to capture both the eye and limb movement of the patient P. As a further illustrative example, a camera that is used to detect eye position and movement can also be used to detect the position and movement of the patient P's limbs.

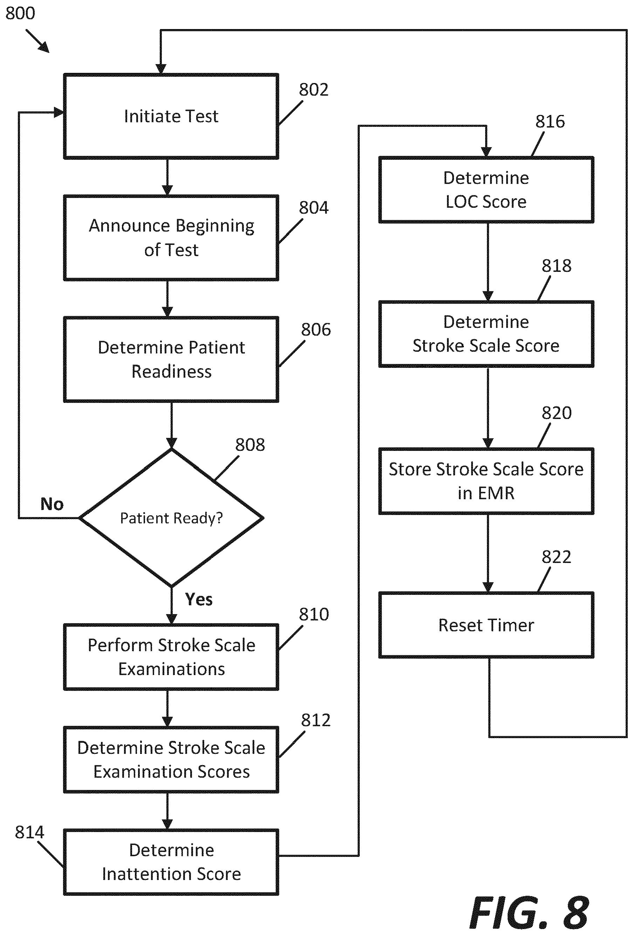

FIG. 8 schematically illustrates a method 800 for determining a stroke scale score for the patient P. The method 800 includes an operation 802 of initiating a test performed by the stroke scale system 100. The initialization can be done by a clinician such as a nurse in the healthcare facility 10. As an illustrative example, a nurse can enter an input to initiate the test at a workstation that is remotely located with respect to the patient P and the stroke scale system 100. Advantageously, the nurse does not need to be physically present in the patient P's room to initiate the test. Alternatively, the initialization at operation 802 can be done automatically by using the timer 114 of the stroke scale system 100 (see FIG. 2). This can be advantageous especially when it is desirable to perform the stroke scale examinations multiple times per day, and the clinicians in the healthcare facility 10 are not available to perform the initialization.

Next, the method 800 includes an operation 804 of announcing the beginning of the test to the patient P. This is helpful to ensure that the patient P is aware that the test is beginning especially when a clinician is not present in the patient P's room. Operation 804 can include providing an audio output from the audio unit 106 to inform the patient P that the test is beginning, and to listen and follow the instructions of the test. Alternatively, or in addition to providing the audio output, operation 804 can include displaying a message on the display unit 104 to inform the patient P that the test is beginning, which can be especially helpful when the patient P has a hearing impairment or is deaf.

Next, the method 800 includes an operation 806 of determining the readiness of the patient P to perform the test. In certain examples, a motion detector 108, such as the body tracker 174, is used to detect the location and position of the patient P such that it can be determined whether the patient P is positioned in the patient support apparatus 102. In certain examples, operation 806 can further include detecting whether the patient P has any amputated limbs or is currently intubated. When it is detected that the patient P is not positioned in the patient support apparatus 102, it is determined that the patient P is not ready to perform the test.

In some instances, operation 806 can include providing an audio output or visual message that requests the patient P to affirmatively confirm whether they are ready to perform the test. Thereafter, the patient P can provide an audible answer (e.g., "Yes" or 'No") that is detected by the microphone 164, or the patient P can enter an answer through the display unit 104 in examples where the display unit 104 is a touchscreen accessible by the patient P.

When the patient P enters an answer that they are not ready to start the test (i.e., "No" at operation 808), the method 800 returns to operation 802 to re-initiate the test after a predetermined amount of time has passed.

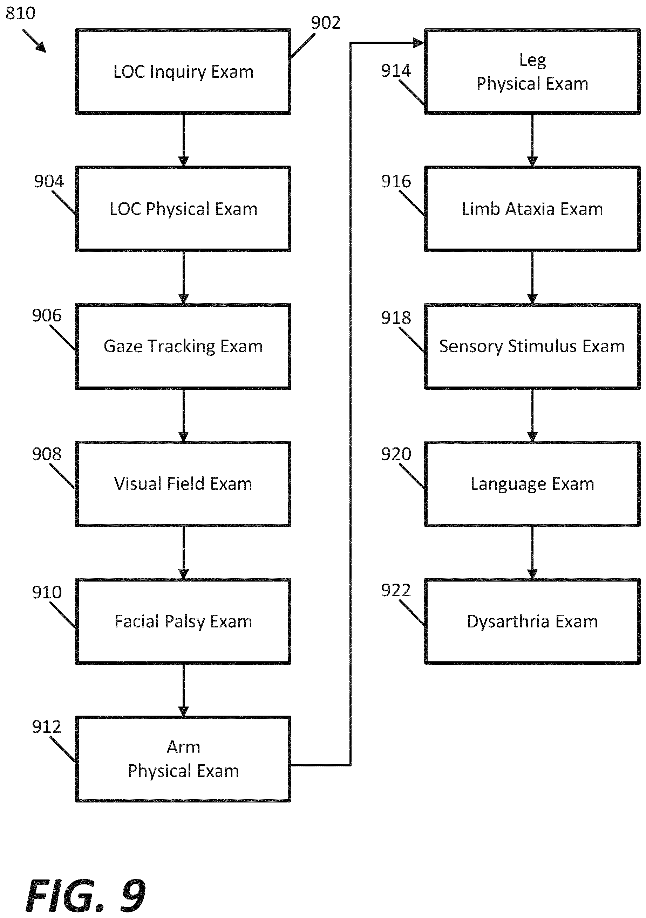

When the patient P enters an answer that they are ready to start the test (i.e., "Yes" at operation 808), the method 800 proceeds to perform a series of stroke scale examinations at operation 810. The stroke scale examinations performed at operation 810 will be described in more detail below with references to FIGS. 9-20.

After completion of the stroke scale examinations, the method 800 proceeds to operation 812 to determine the scores from the stroke scale examinations. In some examples, the score from each stroke scale examination is determined and recorded before proceeding to the next stroke scale examination such that operations 810 and 812 occur simultaneously. Alternatively, the responses from the patient P are recorded during completion of each stroke scale examination, and after completion of all stroke scale examinations, the responses are processed and analyzed such that operation 812 occurs after completion of operation 810. Furthermore, as described above, in some examples, the stroke scale system 100 determines the score from each stroke scale examination, or alternatively, the server 300 can determine the score from each stroke scale examination based on the recorded responses.

Next, the method 800 proceeds to operation 814 to calculate an extinction/inattention score which indicates whether the patient P exhibits a lack of awareness on one side of their body and/or a loss of exploratory search and other actions normally directed toward that side of their body. Data that identifies extinction/inattention is obtained by the stroke scale system 100 during performance of the stroke scale examinations at operation 810 such that this data can be used by the stroke scale system 100 to calculate the extinction/inattention score at operation 814. For example, the data collected from the motion detectors 108 and the microphone 164 of the audio unit 106 can identify whether the patient P exhibited visual, tactile, auditory, spatial, or personal extinction or inattention. Advantageously, the stroke scale system 100 mitigates and/or eliminates clinician subjectivity that is often present when scoring extinction or inattention.

An extinction/inattention score of 0 indicates that there was no abnormality. An extinction/inattention score of 1 is assigned when the data from operation 810 indicates that patient P exhibited visual, tactile, auditory, spatial, or personal extinction or inattention to bilateral simultaneous stimulation of at least one sensory modality. An extinction/inattention score of 2 is assigned when the data from operation 810 indicates that the patient P exhibited extinction or inattention to more than one modality, was orientated to only one side of their body during operation 810 such that they exhibited extinction to bilateral simultaneous stimulation, or exhibited hemi-inattention such that the patient P did not recognize their own hand.

Next, the method 800 proceeds to operation 816 to calculate a level of consciousness (LOC) score for the patient P. Level of consciousness is often not determinable by a few responses to verbal prompts. Thus, the stroke scale system calculates the LOC score based on how well the patient P participated in the other parts of the method 800 (e.g., Was the patient P responsive? Did the patient P follow instructions to the best of their ability and attempt each of the tests? Or did the patient P ignore prompts and not attempt the tests?). For example, data that can identify the patient P's level of consciousness is obtained by the stroke scale system 100 during performance of the stroke scale examinations at operation 810 such that this data can be used by the stroke scale system 100 to calculate the LOC score at operation 816. For example, the data collected from the motion detectors 108 and the microphone 164 of the audio unit 106 can identify whether the patient P was able to follow most of the instructions during operation 810. Advantageously, the stroke scale system 100 mitigates and/or eliminates clinician subjectivity from the calculation of the LOC score.

A LOC score of 0 indicates that the patient P was alert and keenly responsive, such as when the patient P attempts to perform all of the tests. A LOC score of 1 indicates that the patient P was aroused and alert, such as when the patient P attempts more than half of the tests. A LOC score of 2 indicates that the patient P was not alert, such as when the patient P attempts fewer than half of the tests. A LOC score of 3 indicates that the patient P was unresponsive, such as when the patient did not attempt any of the tests.

Next, method 800 proceeds to operation 818 to determine the stroke scale score by combining the stroke scale examination scores (determined at operation 812), the inattention score (determined at operation 814), and the LOC score (determined at operation 816). In certain examples, the stroke scale score determined at operation 818 ranges from 0 to 42 where a score of 0 indicates no stroke, and a score in the range of 21-42 indicates a severe stroke.

In certain examples, when the determined stroke scale score is indicative of a stroke, an alarm is generated to request immediate attention from a caregiver. Example alarms include sounding an alarm at a nurses' station, sounding a local alarm on the patient support apparatus 102, and/or sending an alert message directly to one or more caregivers.

The method 800 further includes an operation 820 of automatically storing the stroke scale score in the EMR 402 of the patient P. Advantageous, operation 820 can be done without any input or intervention from a clinician to reduce human errors that may result from manually updating the EMR 402 of the patient P. Operation 820 is completed by using the network 200 to provide a communications link between the stroke scale system 100 and the server 300, and by the server 300 having access to the EMR system 400.

In some examples, after completion of operation 820, the method includes an operation 822 of resetting the timer 114 to repeat the test after a predetermined period of time has passed. For example, a reminder can be sent to a clinician to repeat the initialization of the test (operation 802) after the predetermined period of time has passed. Alternatively, the timer 114 can instruct the controller 112 to automatically repeat the initialization of the test without requiring any input from a clinician after the predetermined period of time has passed.

FIG. 9 schematically illustrates the stroke scale examinations that are performed at operation 810 in the method 800 for determining the stroke scale score for the patient P. The stroke scale examinations include a level of consciousness (LOC) inquiry exam 902, a level of consciousness (LOC) physical exam 904, a gaze tracking exam 906, a visual field exam 908, a facial palsy exam 910, an arm physical exam 912, a leg physical exam 914, a limb ataxia exam 916, a sensory stimulus exam 918, a comprehension exam 920, and a dysarthria exam 922. In some examples, the stroke scale examinations 902-922 are performed sequentially in the order depicted in FIG. 9. In alternative examples, the stroke scale examinations 902-922 are performed in a different order than the one depicted in FIG. 9.

FIG. 10 schematically illustrates details of the LOC inquiry exam 902. As shown in FIG. 10, the LOC inquiry exam 902 includes an operation 1002 of asking the patient P one or more questions. In some examples, the questions are provided to the patient P by an audio output from the speakers 162 of the audio unit 106. Alternatively, or in addition to providing an audio output, the stroke scale system 100 may also provide the questions to the patient P by displaying a visual message or text on the visual display 142 of the display unit.

The questions may include asking the patient P the month and his or her age. While the foregoing questions are provided as illustrative examples, it is contemplated that are variety of questions may be asked to the patient P during the LOC inquiry exam.

Next, the LOC inquiry exam 902 includes an operation 1004 of receiving the responses from the patient P. In some examples, the responses are audible responses that are recorded by the microphone 164 of the audio unit 106. Alternatively, or in addition to recording an audible response, the stroke scale system 100 may also allow the patient P to type or otherwise enter the answers to the questions using the touch input 144 of the display unit 104 in examples where the display unit 104 is a touchscreen that is accessible by the patient P.

The LOC inquiry exam 902 includes an operation 1006 of processing and recording the responses from the patient P. As described above, the responses from the patient P are used by the stroke scale system 100 or server 300 to calculate the LOC score at operation 816 after completion of the stroke scale examinations 902-922 (i.e., after operation 810 in the method 800). When calculating the LOC score, the recorded responses from the patient P must be correct such that there is no partial credit for the patient P being almost correct.

FIG. 11 schematically illustrates details of the LOC physical exam 904. As shown in FIG. 11, the LOC physical exam 904 includes an operation 1102 of providing a first command to the patient P. In some examples, the first command requests the patient P to open and close their eyes. The stroke scale system 100 provides the first command by an audio output from the audio unit 106, or by a message or text displayed on the display unit 104, or both.

Next, the LOC physical exam 904 includes an operation 1104 of determining whether a response to the first command is detected from the patient P. In examples where the first command requests the patient P to open and close their eyes, the response is detected by using a motion detector 108 such as the eye tracker 172 to detect eye movement. The eye tracker 172 can include a camera that acquires images of the patient P's eyes from which eyelid movement can be detected. Alternatively, a body tracker 174 that uses radar technology can be used to detect eyelid movement to determine whether the patient P opened and closed their eyes.

Next, the LOC physical exam 904 includes an operation 1106 of providing a second command to the patient P. In some examples, the second command requests the patient P to grasp a siderail 90 of the patient support apparatus 102, or a separate device that may be attached or may not be attached to the patient support apparatus 102. The stroke scale system 100 provides the second command by an audio output, or displayed message or text, or both.

Next, the LOC physical exam 904 includes an operation 1108 of determining whether a response to the second command is detected from the patient P. In examples where the second command requests the patient P to grab a siderail 90 of the patient support apparatus 102, the response to the second command is detected by using the one or more pressure sensors 130 provided on the siderail 90, or on a separate device that may be attached or may not be attached to the patient support apparatus 102. Alternatively, a body tracker 174 that uses radar technology can be used to detect whether the patient P grabbed the correct siderail 90 of the patient support apparatus 102, or the correct separate device.

The LOC physical exam 904 includes an operation 1110 of processing and recording the responses from the patient P. In some examples, the stroke scale system 100 calculates a score at operation 1110 based on the recorded responses to the first and second commands. Alternatively, the score can be calculated by the stroke scale system 100 or server 300 after completion of the stroke scale examinations 902-922.

Credit for calculating the LOC command score is given when an unequivocal attempt is made by the patient P to perform a command, but the patient P is not able to complete the command due to weakness. A score of 0 is given when the patient P completes both commands correctly, a score of 1 is given when the patient P completes only one command correctly, and a score of 2 is given when the patient P is not able to complete either command correctly.

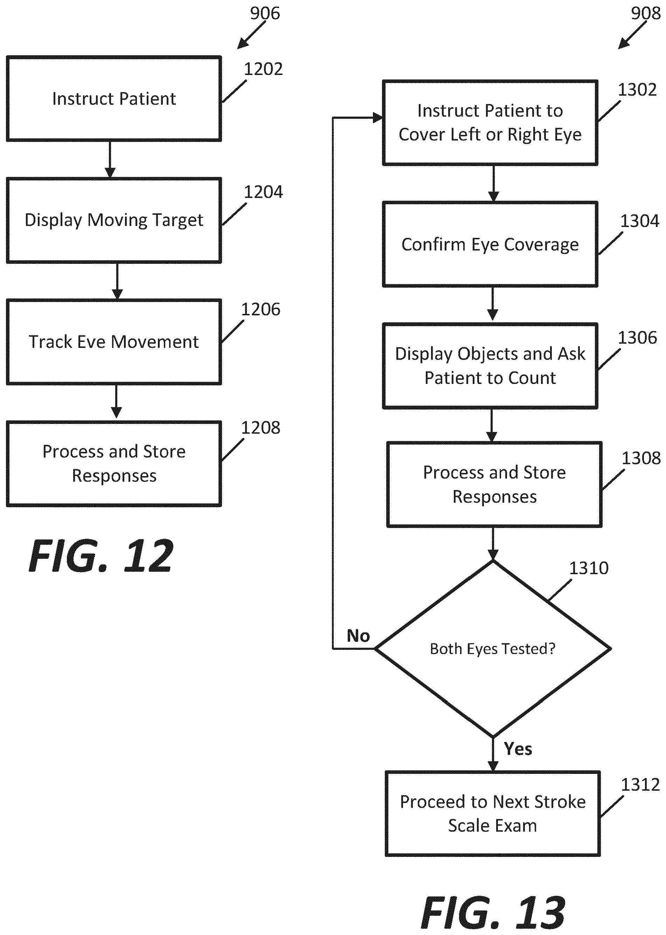

FIG. 12 schematically illustrates details of the gaze tracking exam 906. As shown in FIG. 12, the gaze tracking exam 906 includes an operation 1202 of instructing the patient P to follow a moving target with their eyes. The stroke scale system 100 can provide the instruction to the patient P by an audio output, or displayed text, or both. Thereafter, the gaze tracking exam 906 includes an operation 1204 of displaying the moving target on the display unit 104.

Next, the gaze tracking exam 906 includes an operation 1206 of tracking the eye movement of the patient P while the moving target is displayed on the display unit 104. The stroke scale system tracks the patient P's eye movement by using the eye tracker 172. As discussed above, the eye tracker 172 can include a camera that acquires images of the patient P's eyes from which eye position and movement are extracted.

Thereafter, the gaze tracking exam 906 includes an operation 1208 of processing and recording the patient P's eye movement. In some examples, the stroke scale system 100 calculates a gaze score at operation 1208 based on the recorded eye movement. Alternatively, the gaze score can be calculated by the stroke scale system 100 or server 300 after completion of the stroke scale examinations 902-922 (i.e., at operation 812).

A gaze score of 0 is assigned when the recorded eye movement does not exhibit any abnormality. A gaze score of 1 is assigned when partial gaze palsy is detected from the recorded eye movement, and a gaze score of 2 is assigned when total gaze palsy is detected.

FIG. 13 schematically illustrates details of the visual field exam 908. As shown in FIG. 13, the visual field exam 908 includes an operation 1302 of instructing the patient P to cover their left or right eye with their hand. The stroke scale system 100 can provide the instruction to the patient P by an audio output, or displayed text, or both.

The visual field exam 908 includes an operation 1304 of confirming whether the patient P has covered the correct eye with their hand. The stroke scale system 100 can use a motion detector 108 to detect whether the patient P has covered the correct eye. For example, the body tracker 174 can be used to detect that the patient P has not covered any eye, or has covered an incorrect eye such as when the instruction requests the patient P to cover their left eye, and instead, the patient P incorrectly covers their right eye.

In certain examples, when the motion detector 108 detects that the patient P has not covered an eye, or has covered an incorrect eye, the stroke scale system 100 can repeat the instruction, and/or alert the patient P about the error so that the patient P can correct it to obtain compliance with the instruction. The stroke scale system 100 can repeat the instruction to the patient P by an audio output, or displayed text, or both.

The visual field exam 908 includes an operation 1306 of displaying objects on the display unit 104 and instructing the patient P to count and audibly state the number of objects displayed on the display unit 104 while the left or right eye of the patient P remains covered. The stroke scale system 100 provides the instructions to the patient P by audio output, or displayed text, or both. The stroke scale system 100 determines whether the eye of the patient P remains covered by using a motion detector 108 such as the body tracker 174.

Next, the visual field exam 908 includes an operation 1308 of processing and storing the patient P's responses. For example, the stroke scale system 100 can use the microphone 164 to record the patient P's audible answer to counting the number of objects displayed on the display unit 104 while the patient P's hand remains covering one eye. A score of 0 is assigned when there is no visual loss due the patient P's eye being covered, a score of 1 is assigned when there is partial hemianopia (i.e., blindness in one half of the visual field of the eye), a score of 2 is assigned when there is complete hemianopia in one eye, and a score of 3 is assigned when there is bilateral hemianopia (i.e., hemianopia is present in both eyes).

The visual field exam 908 determines at operation 1310 whether both eyes of the patient P have been examined. When it is determined that both eyes have not been examined (i.e., "No" at operation 1310), the visual field exam 908 repeats operations 1302-1308 for the opposite eye of the patient P. When it is determined that both eyes have been tested (i.e., "Yes" at operation 1310), the stroke scale system 100 proceeds to the next stroke scale examination at operation 1312 such as, for example, the facial palsy exam 910.

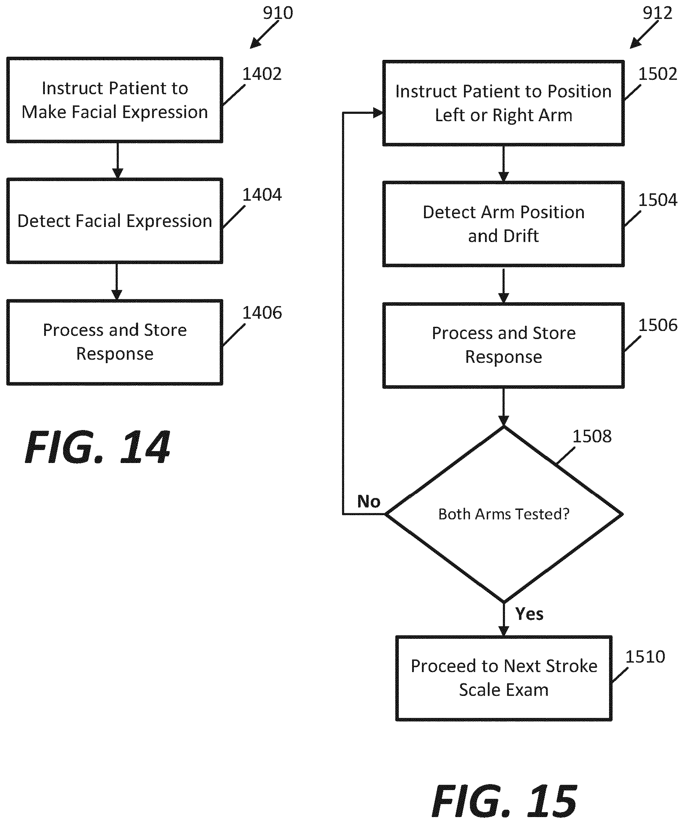

FIG. 14 schematically illustrates details of the facial palsy exam 910. As shown in FIG. 14, the facial palsy exam 910 includes an operation 1402 of instructing the patient P to make a facial expression. The stroke scale system 100 provides the instruction to make a facial expression by an audio output, or displayed message, or both. In some examples, an image or pantomime is displayed on the display unit 104 to encourage the patient P to make a facial expression. Examples of facial expressions may include, without limitation, requesting the patient P to show their teeth, or raise their eyebrows, or close their eyes.

Next, the facial palsy exam 910 includes an operation 1404 of detecting the facial expression. The stroke scale system 100 uses a motion detector 108 such as the body tracker 174 that uses radar or captured image technology to detect the facial expression.

Next, the facial palsy exam 910 includes an operation 1406 of processing and storing the patient P's facial expressions. For example, images of the facial expressions are captured using a camera, and are stored by the stroke scale system 100. The stroke scale system 100 calculates a facial palsy score at operation 1406 based on the captured facial expressions. The facial palsy score can be calculated by the stroke scale system 100 or server 300 after completion of the stroke scale examinations 902-922 (i.e., at operation 812).

A facial palsy score of 0 is assigned when no abnormalities are detected such as when the facial expression is symmetrical on both sides of the patient P's face. A facial palsy score of 1 is assigned when minor paralysis is detected (e.g., an asymmetrical smile), a facial palsy score of 2 is assigned when partial paralysis is detected on one side of the patient P's face, and a facial palsy score of 3 is assigned when complete paralysis is detected on one or both sides of patient P's face such as when there is no facial movement in the upper and lower portions of the face.

FIG. 15 schematically illustrates details of the arm physical exam 912. As shown in FIG. 15, the arm physical exam 912 includes an operation 1502 of instructing the patient P to move their left or right arm into a position, and to maintain the position for a predetermine period of time. For example, the stroke scale system 100 can instruct the patient P to extend their left or right arm (with the palms facing downward) 90 degrees when the patient P is sitting upright, or to extend their left or right arm 45 degrees when the patient P is supine on the patient support apparatus 102, and to maintain the position for 10 seconds.

The stroke scale system 100 provides the instructions by an audio output, or displayed message, or both. As an illustrative example, the stroke scale system 100 can generate an audio output from the audio unit 106 to instruct the patient P to move their left or right arm into the correct position, and can display an image of the correct position on the display unit 104 for visual reference. Additionally, the stroke scale system 100 can display a countdown clock on the display unit 104 to inform the patient P how much longer the patient P is required to maintain the position. Alternatively, or in addition to the countdown clock displayed on the display unit 104, the audio unit 106 can audibly countdown the time remaining.

Next, the arm physical exam 912 includes an operation 1504 of detecting whether the patient P is able to move their arm to the correct position, and thereafter keep the arm in the correct position for a predetermined period of time (e.g., 10 seconds). The stroke scale system 100 uses a motion detector 108, such as the body tracker 174 that uses radar or captured image technology, to detect the position and movement of the patient P's arm.

Next, the arm physical exam 912 includes an operation 1506 of processing and storing the position and movement of the patient P's arm. For example, images of the patient P's arm (e.g., captured by a camera) or data identifying the position and movement of the arm (e.g., determined by a radar system) are processed stored by the stroke scale system 100. In some examples, the stroke scale system 100 calculates an arm physical exam score at operation 1506. Alternatively, the arm physical exam score can be calculated by the stroke scale system 100 or server 300 after completion of the stroke scale examinations 902-922.

An arm physical exam score of 0 is assigned when no drift is detected such as when the arm holds at 90 or 45 degrees for the full 10 seconds. An arm physical exam score of 1 is assigned when minor drift is detected such as when the arm holds at 90 or 45 degrees, but drifts down before the full 10 seconds without hitting the bed or other support. An arm physical exam score of 2 is assigned when it is detected that the arm cannot get to or maintain the 90 or 45 degree angle, drifts down to the bed or other support, but has some effort against gravity. An arm physical exam score of 3 is assigned when the arm immediately falls and there is no effort against gravity. An arm physical exam score of 4 is assigned when no arm movement is detected.

The arm physical exam 912 includes an operation 1508 of determining whether both arms of the patient P have been examined. When it is determined that both arms have not been examined (i.e., "No" at operation 1508), the arm physical exam 912 repeats operations 1502-1506 for the opposite arm. When it is determined that both arms have been tested (i.e., "Yes" at operation 1508), the stroke scale system 100 proceeds to the next stroke scale examination at operation 1510. As an example, the next stroke scale examination is the leg physical exam 914.

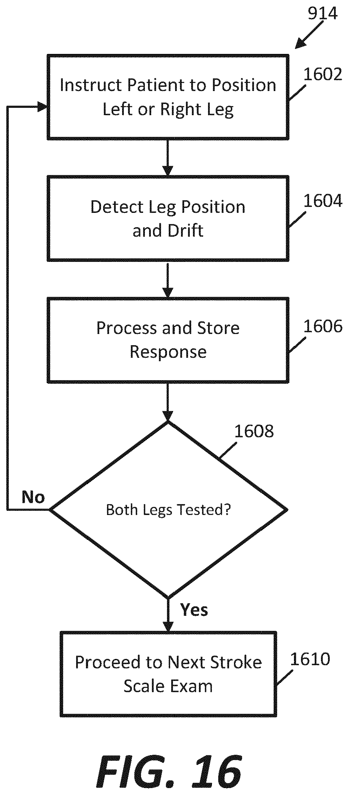

FIG. 16 schematically illustrates details of a leg physical exam 914. As shown in FIG. 16, the leg physical exam 914 includes an operation 1602 of instructing the patient P to move their left or right leg into a position for a predetermined period of time. For example, the patient P can be instructed to lift their left or right leg 30 degrees while they are supine on the patient support apparatus 102, and to maintain the position for 5 seconds.

The stroke scale system 100 provides the instructions by an audio output, or displayed message, or both. As an illustrative example, the stroke scale system 100 generates an audio output from the audio unit 106 to instruct the patient P to move their left or right leg into the correct position, and an image of the correct position can be displayed on the display unit 104 for visual reference. Additionally, the stroke scale system 100 can display a countdown clock on the display unit 104 to inform the patient P for how much longer they are required to maintain the position. Alternatively, or in addition to the countdown clock displayed on the display unit 104, the audio unit 106 can audibly countdown the time remaining.

Next, the leg physical exam 914 includes an operation 1604 of detecting whether the patient P is able to move their leg to the correct position, and thereafter keep the leg in the correct position for a predetermined period of time (e.g., 5 seconds). The stroke scale system 100 uses a motion detector 108, such as the body tracker 174 that uses radar or captured image technology, to detect the position and movement of the patient P's leg.

Next, the leg physical exam 914 includes an operation 1606 of processing and storing the position and movement of the patient P's leg. For example, images of the patient P's leg (e.g., captured by a camera) or data identifying the position and movement of the leg (e.g., determined by a radar system) are processed stored by the stroke scale system 100. In some examples, the stroke scale system 100 calculates a leg physical exam score at operation 1606. Alternatively, the leg physical exam score can be calculated by the stroke scale system 100 or server 300 after completion of the stroke scale examinations 902-922 (i.e., at operation 812).

A leg physical exam score of 0 is assigned when no drift is detected such as when the leg holds at 30 degrees for the full 5 seconds. A leg physical exam score of 1 is assigned when minor drift is detected such as when the leg falls before the full 5 seconds, but does not hit the bed. A leg physical exam score of 2 is assigned when it is detected that the leg falls to the bed before the 5 seconds, but exhibits some effort against gravity. A leg physical exam score of 3 is assigned when the leg immediately falls to the bed and there is no effort against gravity. A leg physical exam score of 4 is assigned when no leg movement is detected.

The leg physical exam 914 includes an operation 1608 of determining whether both legs of the patient P have been examined. When it is determined that both legs have not been examined (i.e., "No" at operation 1608), the leg physical exam 914 repeats operations 1602-1606 for the opposite leg. When it is determined that both legs have been tested (i.e., "Yes" at operation 1608), the stroke scale system 100 proceeds to the next stroke scale examination at operation 1610. As an example, the next stroke scale examination is the limb ataxia exam 916.

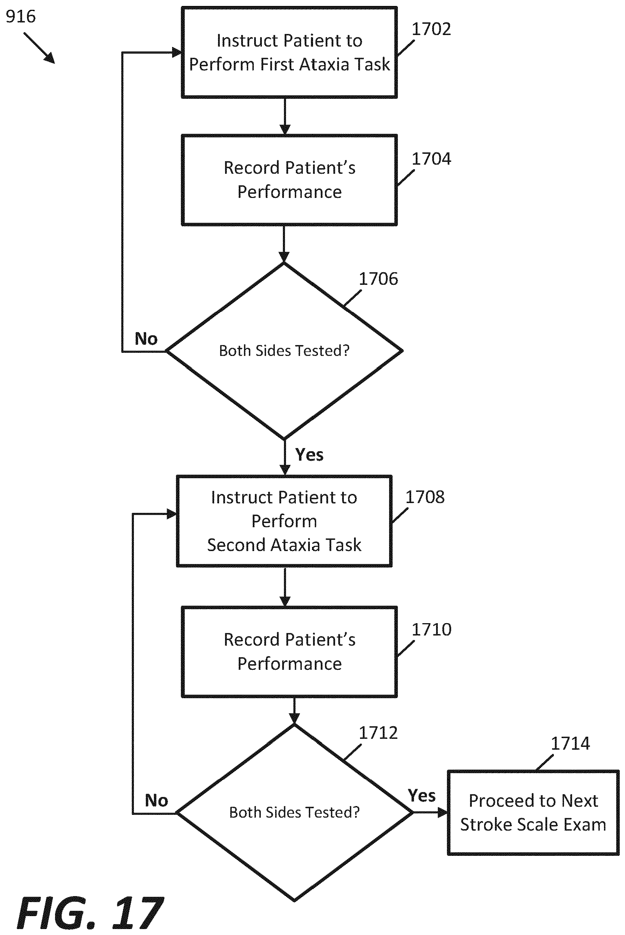

FIG. 17 schematically illustrates details of the limb ataxia exam 916. Limb ataxia is the inability to make smooth, coordinated movements of an arm or a leg, such as when a patient tries to touch their nose with their index finger or when the patient tries to run their right or left heel straight down the opposite shin. The limb ataxia exam 916 includes an operation 1702 of instructing the patient P to perform a first ataxia task. In certain examples, the first ataxia task is a finger-nose-finger test that requires the patient P to extend the index finger of their right or left hand, touch their nose with the same index finger, and then touch the index finger of the opposite hand with the same index finger.

The stroke scale system 100 provides the instructions by an audio output, or displayed message, or both. As an illustrative example, the stroke scale system can generate an audio output from the audio unit 106 to instruct the patient P to extend the index finger of their right or left hand, touch their nose with the same index finger, and then touch the index finger of the opposite hand with the same index finger. A video of the first ataxia task can be displayed on the display unit 104 for visual reference.

Next, the limb ataxia exam 916 includes an operation 1704 of recording and processing the patient P's performance of the first ataxia task. In certain examples, the stroke scale system 100 uses a body tracker 174 that uses radar technology to objectively measure the patient P's performance of the first ataxia task. Alternatively, or in addition to using radar technology, a video of the patient P's performance of the first ataxia task can be recorded.

Next, the limb ataxia exam 916 includes an operation 1706 of determining whether the first ataxia task has been performed for both sides of the patient P's body. When it is determined that both sides have not been examined (i.e., "No" at operation 1706), the limb ataxia exam 916 repeats operations 1702 and 1704 for the opposite side of the patient's body. When the first ataxia task is the finger-nose-finger test, the limb ataxia exam 916 instructs the patient P to use the index finger of the opposite hand to perform the finger-nose-finger test.

When it is determined that both sides have been tested (i.e., "Yes" at operation 1706), the stroke scale system 100 proceeds to operation 1708 which includes instructing the patient P to perform a second ataxia task. In certain examples, the second ataxia task is a heel-shin test that requires the patient P to run their right or left heel straight down the shin of the opposite leg. While the first ataxia task is described as the finger-nose-finger test and the second ataxia task is described as the heel-shin test, it is possible for the order of the ataxia tasks to be reversed such that in certain examples, the first ataxia task is the heel-shin test and the second ataxia task is the finger-nose-finger test.

The limb ataxia exam 916 includes an operation 1710 of recording and processing the patient P's performance of the second ataxia task. In certain examples, the stroke scale system 100 uses a body tracker 174 that uses radar technology to objectively measure the patient P's performance of the second ataxia task. Alternatively, or in addition to using radar technology, a video of the patient P's performance of the second ataxia task can be recorded.

Next, the limb ataxia exam 916 includes an operation 1712 of determining whether the second ataxia task has been performed for both sides of the patient P's body. When it is determined that both sides have not been examined (i.e., "No" at operation 1712), the limb ataxia exam 916 repeats operations 1708 and 1710 for the opposite side of the patient's body. For example, in examples where the second ataxia task is the heel-shin test, the limb ataxia exam 916 instructs the patient P to use the heel of the opposite foot to perform the heel-shin test. When it is determined that both sides have been tested (i.e., "Yes" at operation 1712), the stroke scale system 100 proceeds to the next stroke scale examination at operation 1714. As an illustrative example, the next stroke scale examination is the sensory stimulus exam 918.

In some examples, the stroke scale system 100 calculates a limb ataxia score before proceeding to operation 1714 and performing the next stroke scale examination. Alternatively, the limb ataxia score can be calculated by the stroke scale system 100 or server 300 after completion of the stroke scale examinations 902-922 (i.e., at operation 812).

A limb ataxia score of 0 is given when no ataxia is detected. A limb ataxia score of 1 is given when ataxia is detected in one limb. A limb ataxia score of 2 is given when ataxia is detected in both limbs. A limb ataxia score is not given for an amputated limb.

FIG. 18 schematically illustrates details of the sensory stimulus exam 918. At operation 1802, the sensory stimulus exam 918 includes announcing the start of the sensory stimulus exam so that the patient P is prepared and is not startled by the stimuli that are provided during the exam. The stroke scale system 100 can announce the start of the sensory stimulus exam by an audio output, or displayed text, or both.

Next, the sensory stimulus exam 918 includes an operation 1804 of projecting a probe to provide a sensory stimulus that can be felt by the patient P such as by gently poking or prodding the patent P without harming the patient P. As described above, one or more probes 132 are embedded in a portion of the patient support apparatus 102 such as in a siderail of a hospital bed or an armrest of a chair. In certain examples, the probes 132 are stored inside cavities of a siderail 90, and are controlled by the controller 112 to project outwardly to provide the sensory stimulus during the sensory stimulus exam 918. In alternative examples, the probes 132 can be embedded in a device that is separate from the patient support apparatus 102, and that may or may not attach to the patient support apparatus 102.

The sensory stimulus exam 918 includes an operation 1806 of detecting a response from the patient P to the sensory stimulus. For example, a motion detector 108, such as the body tracker 174 that uses radar or captured image technology, is used by the stroke scale system 100 to detect a facial expression such as a grimace that is generated by the patient P in response to the sensory stimulus. Alternatively, or in addition to using the body tracker 174, the stroke scale system 100 can provide an audio output or visual message that requests the patient P to confirm whether they felt the sensory stimulus. Thereafter, the patient P can provide an audible answer detectible by the microphone 164, or the patient P can enter an answer through the display unit 104 in examples where the display unit 104 is a touchscreen.

Next, the sensory stimulus exam 918 includes an operation 1808 of processing and storing the patient P's response to the sensory stimulus. In some examples, operations 1804 and 1806 are repeated for multiple probes that each provide a different type or intensity of stimulus. For example, operations 1804 and 1806 can be performed for each of the probes 132a-132c depicted in FIG. 24 such that the sensory stimulus exam 918 provides different types of sensory stimuli, and records the patient P's responses to each type of stimuli. Additionally, operations 1804 and 1806 can be repeated for stimuli that are provided to different portions of the patient P's body such as the arms, legs, torso, and the like, and for different sides of the patient P's body such as the right side and the left side of the body.

In some examples, the stroke scale system 100 calculates a sensory score at operation 1808 based on the detection of patient P's responses to the sensory stimuli. Alternatively, the sensory score is calculated by the stroke scale system 100 or server 300 after completion of the stroke scale examinations 902-922 (i.e., at operation 812).

A sensory score of 0 is assigned when no sensory loss is detected. A sensory score of 1 is assigned when there is moderate sensory loss such as when the patient P feels the sensory stimuli, but the feeling is less sharp or is duller than what should ordinarily be felt by normal patients. A sensory score of 2 is assigned when the patient P cannot sense being touched at all.

FIG. 19 schematically illustrates details of the comprehension exam 920. The comprehension exam 920 is performed to detect aphasia which is a disorder that affects a person's ability to express and understand written and spoken language. Aphasia can occur suddenly after a stroke or head injury, or develop slowly from a growing brain tumor.

At operation 1902, the comprehension exam 920 includes instructing the patient P to view the display unit 104. The stroke scale system 100 can provide the instructions at operation 1902 by an audio output, or displayed text, or both.

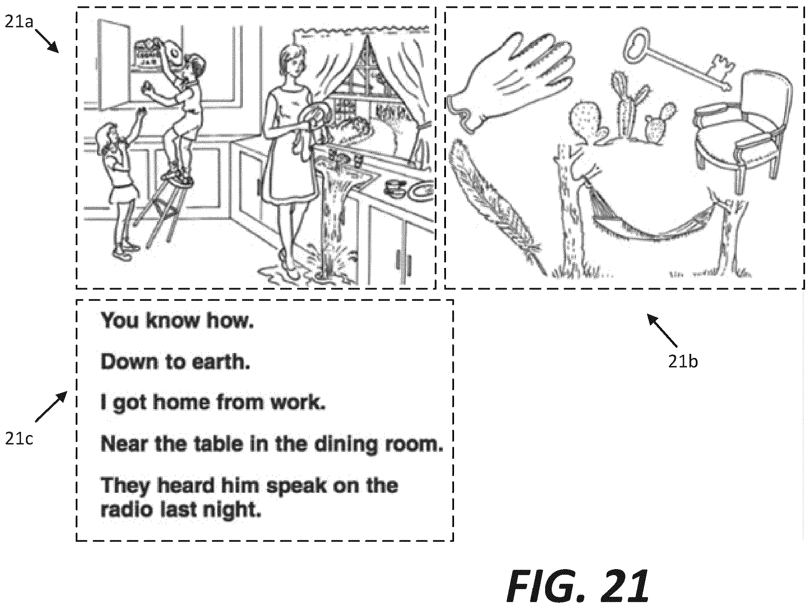

Next, the comprehension exam 920 includes an operation 1904 of displaying an image or video on the display unit 104. As an illustrative example, FIG. 21 shows an image 21a, a naming sheet 21b, and a list of sentences 21c that can be displayed on the display unit 104 during operation 1904 of the comprehension exam 920.

Thereafter, the comprehension exam 920 includes an operation 1906 of instructing the patient P to describe the images and videos displayed on the display unit 104. For example, operation 1906 can include asking the patient P to verbally describe what is happening in the image 21a, to name items in the naming sheet 21b, and/or to read from the list of sentences 21c displayed on the display unit 104. When the patient P is blind, operations 1904 and 1906 can include generating audio outputs, and instructing the patient P to repeat the audio outputs.

Next, the comprehension exam 920 includes an operation 1908 of processing and storing the response from the patient P. For example, the responses from the patient P can be recorded using the microphone 164 of the audio unit 106. When the patient P is intubated, the patient P can be asked to enter their responses using the touch input 144 of the display unit 104.

In some examples, the stroke scale system 100 calculates a comprehension score at operation 1908 based on the recorded responses from the patient P. Alternatively, the comprehension score is calculated by the stroke scale system 100 or server 300 after completion of the stroke scale examinations 902-922 (i.e., at operation 812).

A comprehension score of 0 is assigned when no aphasia is detected by the stroke scale system 100. A comprehension score of 1 is assigned when moderate aphasia is detected such as when the patient P exhibits some loss of comprehension without significant limitation such that the image 21a, items in the naming sheet 21b, and/or to the list of sentences 21c can be identified from the patient P's responses. A comprehension score of 2 is assigned when severe aphasia is detected such as when all communication is through fragmentary expression, and the image 21a, items in the naming sheet 21b, and/or to the list of sentences 21c cannot be identified from the patient P's responses. A comprehension score of 3 is assigned when global aphasia is detected such that no comprehension is identified from the patient P's recorded responses.

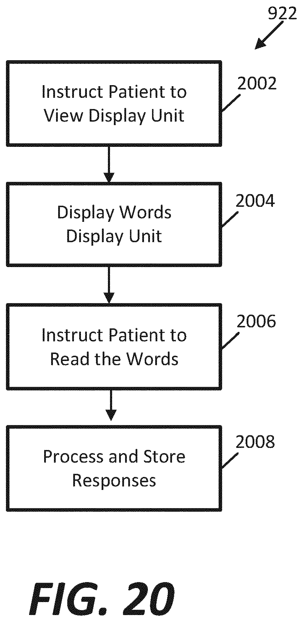

FIG. 20 schematically illustrates details of a dysarthria exam 922. The dysarthria exam 922 is performed to detect dysarthria which is a motor speech disorder in which the muscles that are used to produce speech are damaged, paralyzed, or weakened. The person with dysarthria cannot control his or her tongue, larynx, vocal cords, and surrounding muscles, which makes it difficult for the person to form and pronounce words. Weakness in the muscles used for speech can cause the patient P to have slowed or slurred speech.

The dysarthria exam 922 includes operation 2002 of instructing the patient P to view the display unit 104. The stroke scale system 100 can provide the instructions at operation 2002 by generating an audio output, or displayed text, or both.

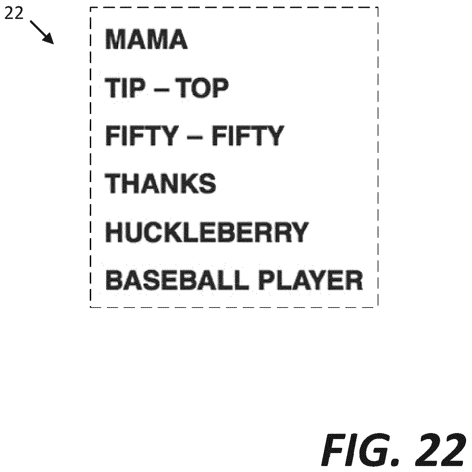

Next, the dysarthria exam 922 includes an operation 2004 of displaying words on the display unit 104. As an illustrative example, FIG. 22 shows words 22 that can be displayed on the display unit 104 during operation 2004 of the dysarthria exam 922.

Thereafter, the dysarthria exam 922 includes an operation 2006 of instructing the patient P to verbally read the words 22 displayed on the display unit 104. When the patient P is blind, operations 2004 and 2006 can include generating audio outputs, and instructing the patient P to verbally repeat the audio outputs such that the speech of the patient P can be obtained.

Next, the dysarthria exam 922 includes an operation 2008 of processing and storing the response from the patient P. For example, the responses from the patient P can be recorded using the microphone 164 of the audio unit 106. In some examples, the stroke scale system 100 calculates a dysarthria score at operation 2008 based on the recorded responses from the patient P. Alternatively, the dysarthria score is calculated by the stroke scale system 100 or server 300 after completion of the stroke scale examinations 902-922 (i.e., at operation 812).

A dysarthria score of 0 is assigned when no dysarthria is detected such that the patient P's speech is normal. A dysarthria score of 1 is assigned when moderate dysarthria is detected such as when the patient P slurs at least some of the words 22, and can be understood with some difficulty. A dysarthria score of 2 is assigned when severe dysarthria is detected such as when the patient P's speech is so slurred it is unintelligible.

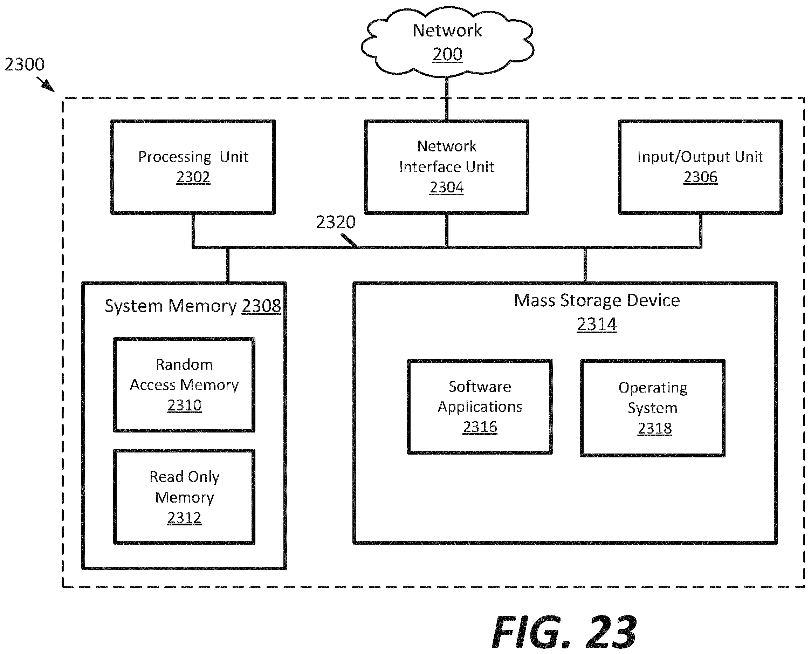

FIG. 23 illustrates an exemplary architecture of a computing device 2300 which can be used to implement aspects of the present disclosure, such as the functions of the stroke scale system 100 described above. The computing device 2300 includes a processing unit 2302, a system memory 2308, and a system bus 2320 that couples the system memory 2308 to the processing unit 2302. The processing unit 2302 is an example of a processing device such as a central processing unit (CPU). The system memory 2308 includes a random-access memory ("RAM") 2310 and a read-only memory ("ROM") 2312. A basic input/output logic containing the basic routines that help to transfer information between elements within the computing device 2300, such as during startup, is stored in the ROM 2312.

The computing device 2300 can also include a mass storage device 2314 that is able to store software instructions and data. The mass storage device 2314 is connected to the processing unit 2302 through the system bus 2320. The mass storage device 2314 and its associated computer-readable data storage media provide non-volatile, non-transitory storage for the computing device 2300.

Although the description of computer-readable data storage media contained herein refers to a mass storage device, it should be appreciated by those skilled in the art that computer-readable data storage media can be any available non-transitory, physical device or article of manufacture from which the device can read data and/or instructions. The mass storage device 2314 is an example of a computer-readable storage device.

Computer-readable data storage media include volatile and non-volatile, removable and non-removable media implemented in any method or technology for storage of information such as computer-readable software instructions, data structures, program modules or other data. Example types of computer-readable data storage media include, but are not limited to, RAM, ROM, EPROM, EEPROM, flash memory or other solid-state memory technology, or any other medium which can be used to store information.

The computing device 2300 may operate in a networked environment using logical connections to remote network devices through the network 200, such as a local network, the Internet, or another type of network. The device connects to the network 200 through a network interface unit 2304 connected to the system bus 2320. The network interface unit 2304 may also connect to other types of networks and remote computing systems.

The computing device 2300 can also include an input/output controller 2306 for receiving and processing input from a number of input devices. Similarly, the input/output controller 2306 may provide output to a number of output devices.

The mass storage device 2314 and the RAM 2310 can store software instructions and data. The software instructions can include an operating system 2318 suitable for controlling the operation of the device. The mass storage device 2314 and/or the RAM 2310 also store software instructions 2316, that when executed by the processing unit 2302, cause the device to provide the functionality of the stroke scale system 100 discussed in this document. For example, the mass storage device 2314 and/or the RAM 2310 can store software instructions that, when executed by the processing unit 2302, cause the stroke scale system 100 to perform the method 800 for determining a stroke scale score for the patient P.

The various examples described above are provided by way of illustration only and should not be construed to be limiting in any way.

## Claims

1. A stroke scale system (100), comprising:
a patient support apparatus (102) having one or more pressure sensors (130) that can be grasped by a patient while the patient is supported on the patient support apparatus (102);
one or more motion detectors (108) that detect movements of the patient;
a display unit (104) positioned relative to the patient support apparatus (102) to be viewable by the patient while the patient is supported on the patient support apparatus (102);
an audio unit (106) having one or more speakers (162) and a microphone (164); and
a controller (112) having at least one processor, and a memory storing instructions which, when executed by the at least one processor, cause the system to:
perform a series of stroke scale examinations by using the display unit (104) or the audio unit (106) to provide instructions to the patient, and using the one or more motion detectors (108) and microphone (164) to determine compliance with the instructions, wherein at least one stroke scale examination instructs the patient to grasp a portion of the patient support apparatus (102), and the pressure sensors (130) determine compliance with the instructions by measuring an applied pressure to the portion of the patient support apparatus (102); and
**characterized in that**
the portion of the patient support apparatus (102) further includes one or more probes (132) stored inside respective cavities in a siderail (90) of the patient support apparatus (102) adjacent to the one or more pressure sensors (130),
and wherein another stroke scale examination includes:
projecting the one or more probes (132) outwardly from the siderail to provide a sensory stimulus,
detecting the patient's response to the sensory stimulus,
returning the one or more probes to inside their respective cavities so that they do not disturb the patient, and
calculate a stroke scale score by combining scores determined from the series of stroke scale examinations.

2. The system (100) of claim 1, wherein the patient support apparatus (102) is a bed, and the pressure sensors (130) are provided on one or more siderails (90) of the bed.

3. The system (100) of claim 1 or claim 2, wherein the one or more probes (132) include a first probe having a blunt distal end that provides a mild sensory stimulus, a second probe having a pointed distal end that provides a moderate sensory stimulus, and a third probe having another pointed distal end that provides a stronger sensory stimulus.

4. The system (100) of any preceding claim, wherein the memory stores further instructions which, when executed by the at least one processor, cause the system to:
after completion of the stroke scale examinations, reset a timer to repeat the stroke scale examinations after a predetermined period of time has passed.

5. A method of determining a stroke scale score, the method comprising:
performing a series of stroke scale examinations by using a display unit (104) or an audio unit (106) to provide instructions to a patient supported on a patient support apparatus (102), at least one stroke scale examination instructing the patient to grasp a portion of the patient support apparatus (102);
using a pressure sensor (130) provided on the portion of the patient support apparatus (102) to determine compliance with the instruction to grasp the portion;
**characterized in that** another stroke scale examination includes:
projecting one or more probes (132) stored inside respective cavities in a siderail of the patient support apparatus (102) adjacent to the pressure sensor (130) outwardly from the siderail to provide a sensory stimulus,
detecting the patient's response to the sensory stimulus,
returning the one or more probes to inside their respective cavities so that they do not disturb the patient, and
calculating a stroke scale score by combining scores determined from each of the stroke scale examinations.

6. The method of claim 5, wherein measuring the patient's response to the sensory stimulus includes using radar or captured image technology to detect a facial expression of the patient that is generated by the patient in response to the sensory stimulus.

7. The method of either claim 6 or claim 7, wherein measuring the patient's response to the sensory stimulus includes receiving an audible answer from the patient detected by a microphone (164).

8. A non-transitory computer readable storage media including computer readable instructions which, when read and executed by a computing device of a patient support apparatus, cause the computing device to:
perform a series of stroke scale examinations by using a display unit (104) or an audio unit (106) to provide instructions to a patient supported on a patient support apparatus (102), at least one stroke scale examination instructing the patient to grasp a portion of the patient support apparatus (102);
use a pressure sensor (130) provided on the portion of the patient support apparatus (102) to determine compliance with the instruction to grasp the portion;
**characterized in that** another stoke scale examination includes:
projecting one or more probes (132) stored inside respective cavities in a siderail of the patient support apparatus (102) adjacent to the pressure sensor (130) outwardly from the siderail to provide a sensory stimulus,
detecting the patient's response to the sensory stimulus,
returning the one or more probes to inside their respective cavities so that they do not disturb the patient, and
calculate a stroke scale score by combining scores determined from each of the stroke scale examinations,
wherein the patient support apparatus comprises the display unit, the audio unit, the pressure sensor, and the one or more probes.

9. The non-transitory computer readable storage media of claim 8, wherein measuring the patient's response to the sensory stimulus includes using radar or captured image technology to detect a facial expression of the patient generated in response to the sensory stimulus.

10. The non-transitory computer readable storage media of claim 9, wherein measuring the patient's response to the sensory stimulus includes receiving an audible answer from the patient detected by a microphone (164).

## Patentansprüche

1. Schlaganfallskalensystem (100), umfassend:
eine Patientenlagerungsvorrichtung (102) mit einem oder mehreren Drucksensoren (130), die von einem Patienten ergriffen werden können, während der Patient auf der Patientenlagerungsvorrichtung (102) gelagert ist;
einen oder mehrere Bewegungsmelder (108), die Bewegungen des Patienten erkennen;
eine Anzeigeeinheit (104), die relativ zur Patientenlagerungsvorrichtung (102) positioniert ist, um vom Patienten anschaubar zu sein, während der Patient auf der Patientenlagerungsvorrichtung (102) gelagert ist;
eine Audioeinheit (106) mit einem oder mehreren Lautsprechern (162) und einem Mikrofon (164); und
eine Steuerung (112) mit mindestens einem Prozessor und einem Speicher, der Anweisungen speichert, die bei Ausführung durch den mindestens einen Prozessor das System veranlassen zum:
Durchführen einer Reihe von Schlaganfallskalenuntersuchungen unter Verwendung der Anzeigeeinheit (104) oder der Audioeinheit (106) zum Geben von Anweisungen an den Patienten und Verwenden des/der einen oder mehreren Bewegungsmelder (108) und des Mikrofons (164), um die Befolgung der Anweisungen zu bestimmen, wobei mindestens eine Schlaganfallskalenuntersuchung den Patienten anweist, einen Teil der Patientenlagerungsvorrichtung (102) zu ergreifen, und die Drucksensoren (130) die Befolgung der Anweisungen bestimmen, indem sie auf den Teil der Patientenlagerungsvorrichtung (102) ausgeübten Druck messen; und
**dadurch gekennzeichnet, dass**
der Teil der Patientenlagerungsvorrichtung (102) ferner eine oder mehrere Sonden (132) aufweist, die im Inneren von jeweiligen Hohlräumen in einem Seitenteil (90) der Patientenlagerungsvorrichtung (102) neben dem/den einen oder mehreren Drucksensoren (130) aufbewahrt werden,
und wobei eine andere Schlaganfalluntersuchung Folgendes aufweist:
Vorbewegen der einen oder mehreren Sonden (132) aus dem Seitenteil nach außen, um einen Sinnesreiz bereitzustellen,
Erkennen der Reaktion des Patienten auf den Sinnesreiz,
Zurückführen der einen oder mehreren Sonden ins Innere ihrer jeweiligen Hohlräume, so dass sie den Patienten nicht stören, und
Berechnen eines Schlaganfallskalenwerts durch Kombinieren von anhand der Reihe von Schlaganfallskalenuntersuchungen bestimmten Werten.

2. System (100) nach Anspruch 1, wobei die Patientenlagerungsvorrichtung (102) ein Bett ist und die Drucksensoren (130) an einem oder mehreren Seitenteilen (90) des Betts bereitgestellt sind.

3. System (100) nach Anspruch 1 oder Anspruch 2, wobei die eine oder mehreren Sonden (132) eine erste Sonde mit einem stumpfen distalen Ende, die einen milden Sinnesreiz bereitstellt, eine zweite Sonde mit einem spitzen distalen Ende, die einen mäßigen Sinnesreiz bereitstellt, und eine dritte Sonde mit einem weiteren spitzen distalen Ende, die einen stärkeren Sinnesreiz bereitstellt, aufweisen.

4. System (100) nach einem der vorhergehenden Ansprüche, wobei der Speicher weitere Anweisungen speichert, die bei Ausführung durch den mindestens einen Prozessor das System veranlassen zum:
nach Abschluss der Schlaganfallskalenuntersuchungen Rücksetzen eines Timers zum Wiederholen der Schlaganfallskalenuntersuchungen nach Ablauf einer vorbestimmten Zeitspanne.

5. Verfahren zum Bestimmen eines Schlaganfallskalenwerts, wobei das Verfahren Folgendes umfasst:
Durchführen einer Reihe von Schlaganfallskalenuntersuchungen durch Verwenden einer Anzeigeeinheit (104) oder einer Audioeinheit (106) zum Geben von Anweisungen an einen Patienten, der auf einer Patientenlagerungsvorrichtung (102) gelagert ist, wobei mindestens eine Schlaganfallskalenuntersuchung den Patienten anweist, einen Teil der Patientenlagerungsvorrichtung (102) zu ergreifen;
Verwenden eines Drucksensors (130), der an dem Teil der Patientenlagerungsvorrichtung (102) bereitgestellt ist, um die Befolgung der Anweisung zum Ergreifen des Teils zu bestimmen;
**dadurch gekennzeichnet, dass** eine andere Schlaganfallskalenuntersuchung Folgendes aufweist:
Vorbewegen von einer oder mehreren Sonden (132), die im Inneren von jeweiligen Hohlräumen in einem Seitenteil der Patientenlagerungsvorrichtung (102) neben dem Drucksensor (130) aufbewahrt werden, aus dem Seitenteil nach außen, um einen Sinnesreiz bereitzustellen,
Erkennen der Reaktion des Patienten auf den Sinnesreiz,
Zurückführen der einen oder mehreren Sonden ins Innere ihrer jeweiligen Hohlräume, so dass sie den Patienten nicht stören, und
Berechnen eines Schlaganfallskalenwerts durch Kombinieren von anhand jeder der Schlaganfallskalenuntersuchungen bestimmten Werten.

6. Verfahren nach Anspruch 5, wobei das Messen der Reaktion des Patienten auf den Sinnesreiz die Verwendung von Radar- oder Bildverarbeitungstechnologie zum Erkennen eines Gesichtsausdrucks des Patienten, der vom Patienten als Reaktion auf den Sinnesreiz erzeugt wird, aufweist.

7. Verfahren nach Anspruch 6 oder Anspruch 7, wobei das Messen der Reaktion des Patienten auf den Sinnesreiz das Empfangen einer durch ein Mikrofon (164) erkannten hörbaren Antwort des Patienten aufweist.

8. Nichtflüchtiges computerlesbares Speichermedium, das computerlesbare Anweisungen aufweist, die bei Lesen und Ausführung durch eine Rechenvorrichtung einer Patientenlagerungsvorrichtung die Rechenvorrichtung veranlassen zum:
Durchführen einer Reihe von Schlaganfallskalenuntersuchungen durch Verwenden einer Anzeigeeinheit (104) oder einer Audioeinheit (106) zum Geben von Anweisungen an einen Patienten, der auf einer Patientenlagerungsvorrichtung (102) gelagert ist, wobei mindestens eine Schlaganfallskalenuntersuchung den Patienten anweist, einen Teil der Patientenlagerungsvorrichtung (102) zu ergreifen;
Verwenden eines Drucksensors (130), der an dem Teil der Patientenlagerungsvorrichtung (102) bereitgestellt ist, um die Befolgung der Anweisung zum Ergreifen des Teils zu bestimmen;
**dadurch gekennzeichnet, dass** eine andere Schlaganfallskalenuntersuchung Folgendes aufweist:
Vorbewegen von einer oder mehreren Sonden (132), die im Inneren von jeweiligen Hohlräumen in einem Seitenteil der Patientenlagerungsvorrichtung (102) neben dem Drucksensor (130) aufbewahrt werden, aus dem Seitenteil nach außen, um einen Sinnesreiz bereitzustellen,
Erkennen der Reaktion des Patienten auf den Sinnesreiz,
Zurückführen der einen oder mehreren Sonden ins Innere ihrer jeweiligen Hohlräume, so dass sie den Patienten nicht stören, und
Berechnen eines Schlaganfallskalenwerts durch Kombinieren von anhand jeder der Schlaganfallskalenuntersuchungen bestimmten Werten,
wobei die Patientenlagerungsvorrichtung die Anzeigeeinheit, die Audioeinheit, den Drucksensor und die eine oder mehreren Sonden umfasst.

9. Nichtflüchtiges computerlesbares Speichermedium nach Anspruch 8, wobei das Messen der Reaktion des Patienten auf den Sinnesreiz die Verwendung von Radar- oder Bildverarbeitungstechnologie zum Erkennen eines Gesichtsausdrucks des Patienten, der als Reaktion auf den Sinnesreiz erzeugt wird, aufweist.

10. Nichtflüchtiges computerlesbares Speichermedium nach Anspruch 9, wobei das Messen der Reaktion des Patienten auf den Sinnesreiz das Empfangen einer durch ein Mikrofon (164) erkannten hörbaren Antwort des Patienten aufweist.

## Revendications

1. Système d'échelle d'évaluation d'accident vasculaire cérébral (AVC) (100) comprenant :
un appareil de support de patient (102) ayant un ou plusieurs capteurs de pression (130) qui peuvent être saisis par un patient pendant que le patient est soutenu sur l'appareil de support de patient (102) ;
un ou plusieurs détecteurs de mouvements (108) qui détectent des mouvements du patient ;
une unité d'affichage (104) positionnée par rapport à l'appareil de support de patient (102) pour pouvoir être vue par le patient pendant que le patient est soutenu sur l'appareil de support de patient (102) ;
une unité audio (106) ayant un ou plusieurs haut-parleurs (162) et un microphone (164) ; et
un contrôleur (112) ayant au moins un processeur, et une mémoire stockant des instructions qui, lorsque exécutées par le au moins un processeur, font que le système :
effectue une série d'examens d'échelle d'AVC en utilisant l'unité d'affichage (104) ou l'unité audio (106) pour donner des instructions au patient, et en utilisant l'un ou les plusieurs détecteurs de mouvements (108) et le microphone (164) pour déterminer l'observance des instructions, dans lequel au moins un examen d'échelle d'AVC donne au patient l'instruction de saisir une partie de l'appareil de support de patient (102), et les capteurs de pression (130) déterminent l'observance des instructions en mesurant une pression appliquée à la partie de l'appareil de support de patient (102) ; et
**caractérisé en ce que**
la partie de l'appareil de support de patient (102) comprend en outre une ou plusieurs sondes (132) stockées à l'intérieur de cavités respectives dans une barrière latérale (90) de l'appareil de support de patient (102) adjacentes à l'un ou plusieurs capteurs (130),
et dans lequel un autre examen d'échelle d'AVC comprend :
faire saillir l'une ou plusieurs sondes (132) vers l'extérieur de la barrière latérale pour donner un stimulus sensoriel,
détecter la réponse du patient au stimulus sensoriel,
remettre l'une ou plusieurs sondes à l'intérieur de leur cavité respective pour qu'elles ne gênent pas le patient, et
calculer un score d'échelle d'AVC en combinant les scores déterminés de la série d'examens d'échelle d'AVC.

2. Système (100) selon la revendication 1, dans lequel l'appareil de support de patient (102) est un lit, et les capteurs de pression (130) sont disposés sur une ou plusieurs barrières latérales (90) du lit.

3. Système (100) selon la revendication 1 ou la revendication 2, dans lequel l'une ou les plusieurs sondes (132) comprennent une première sonde ayant une extrémité distale émoussée qui donne un stimulus sensoriel léger, une deuxième sonde ayant une extrémité distale pointue qui donne un stimulus sensoriel modéré, et une troisième sonde ayant une autre extrémité distale pointue qui donne un stimulus sensoriel plus fort.

4. Système (100) selon l'une quelconque des revendications précédentes, dans lequel la mémoire stocke des instructions supplémentaires qui, lorsque exécutées par le au moins un processeur, font que le système :
après que les examens d'échelle d'AVC ont été complétés, remet une horloge à l'état initial afin de répéter les examens d'échelle d'AVC après qu'une période de temps prédéterminée s'est écoulée.

5. Procédé de détermination de score d'échelle d'AVC, le procédé comprenant :
effectuer une série d'examens d'échelle d'AVC en utilisant une unité d'affichage (104) ou une unité audio (106) pour donner des instructions à un patient soutenu sur un appareil de support de patient (102), au moins un examen d'échelle d'AVC donnant au patient l'instruction de saisir une partie de l'appareil de support de patient (102) ;
utiliser un capteur de pression (130) disposé sur la partie de l'appareil de support de patient (102) afin de déterminer l'observance de l'instruction de saisir la partie ;
**caractérisé en ce qu'**un autre examen d'échelle d'AVC comprend :
faire saillir une ou plusieurs sondes (132) stockées à l'intérieur de cavités respectives dans une barrière latérale de l'appareil de support de patient (102) adjacentes au capteur de pression (130), vers l'extérieur de la barrière latérale pour donner un stimulus sensoriel,
détecter la réponse du patient au stimulus sensoriel,
remettre la ou les plusieurs sondes à l'intérieur de leur cavité respective pour qu'elles ne gênent pas le patient, et
calculer un score d'échelle d'AVC en combinant les scores déterminés pour chacun des examens d'échelle d'AVC.

6. Procédé selon la revendication 5, dans lequel mesurer la réponse du patient au stimulus sensoriel comprend utiliser une technologie radar ou d'image saisie pour détecter une expression faciale du patient qui est générée par le patient en réponse au stimulus sensoriel.

7. Procédé selon la revendication 6 ou la revendication 7, dans lequel mesurer la réponse du patient au stimulus sensoriel comprend recevoir une réponse audible du patient détectée par un microphone (164).

8. Support de stockage non transitoire lisible par ordinateur comprenant des instructions lisibles par ordinateur qui, lorsque lues et exécutées par un dispositif informatique d'un appareil de support de patient, font que le dispositif informatique :
effectue une série d'examens d'échelle d'AVC en utilisant une unité d'affichage (104) ou une unité audio (106) pour donner des instructions à un patient soutenu sur un appareil de support de patient (102), au moins un examen d'échelle d'AVC donnant au patient l'instruction de saisir une partie de l'appareil de support de patient (102) ;
utilise un capteur de pression (130) disposé sur la partie de l'appareil de support de patient (102) afin de déterminer l'observance de l'instruction de saisir la partie ;
**caractérisé en ce qu'**un autre examen d'échelle d'AVC comprend :
faire saillir une ou plusieurs sondes (132) stockées à l'intérieur de cavités respectives dans une barrière latérale de l'appareil de support de patient (102) adjacentes au capteur de pression (130), vers l'extérieur de la barrière latérale pour donner un stimulus sensoriel,
détecter la réponse du patient au stimulus sensoriel,
remettre la ou les plusieurs sondes à l'intérieur de leur cavité respective pour qu'elles ne gênent pas le patient, et
calculer un score d'échelle d'AVC en combinant les scores déterminés pour chacun des examens d'échelle d'AVC,
où l'appareil de support de patient comprend l'unité d'affichage, l'unité audio, le capteur de pression et l'une ou les plusieurs sondes.

9. Support de stockage non transitoire lisible par ordinateur selon la revendication 8, dans lequel mesurer la réponse du patient au stimulus sensoriel comprend utiliser une technologie radar ou d'image saisie pour détecter une expression faciale du patient générée par le patient en réponse au stimulus sensoriel.

10. Support de stockage non transitoire lisible par ordinateur selon la revendication 9, dans lequel mesurer la réponse du patient au stimulus sensoriel comprend recevoir une réponse audible du patient détectée par un microphone (164).
